# EUROPEAN PATENT APPLICATION

(11) **EP 1 695 742 A1**
(43) Date of publication of application: **30.08.2006**
(21) Application number: 05075885.3
(22) Date of filing: 18.04.2005
(51) Int. Cl.: A61Q 19/00, A61K 8/02

(54) **Line up of wipes products incorporating targeted sensory elements**

(30) Priority: 25.02.2005 US 66091
(71) Applicant: THE PROCTER & GAMBLE COMPANY, Cincinnati, Ohio 45202 (US)
(72) Inventor: Norman, Joshua James, 45202 Cincinnati Ohio (US)
(74) Representative: Hirsch, Uwe Thomas M.H.

(57) **Abstract**

Line ups of wipes products are disclosed. In particular baby wet wipe line ups having targeted sensory elements are disclosed. The line ups may have targeted scent elements, targeted visual elements, targeted tactile elements and targeted auditory elements. The sensory elements may be provided in combination to reinforce each other or to provide a synergistic effect. Tailoring of the sensory elements may be done on the basis of desired benefit, use situation, stage of development, or any combination of these.

## Description

### FIELD OF THE INVENTION

This invention relates to disposable wipes products. In particular, this invention relates to a line up of wet wipe products which are tailored and targeted to groups of consumers on the basis one or more of use situation, desired benefit, or stage of development of the user. The tailoring is accomplished through a combination of specifically adapted sensory elements such as scent elements, visual elements, tactile elements, and auditory elements. These adapted sensory elements may be provided on the wipe substrate, the lotion, the packaging, or any combination of these.

### BACKGROUND OF THE INVENTION

The field of disposable consumer products includes a wide variety of products often referred to generically as "wipes" or "wet-wipes." Typically, these products in their simplest form consist of a substrate, such a web of nonwoven material (often pre-cut into discrete sheets), which has been impregnated with an aqueous lotion (such a cleansing solution or emulsion or paste). Wipes or wet-wipes products are currently sold commercially for a wide variety of consumer applications. These include, cosmetic removal (cosmetic or make-up wipes), feminine hygiene, general face and hand cleaning, household cleaning (such as bleach impregnated wipes), automobile cleaning, and wiping of babies and children such as during a diaper change. In this last category, there are a wide variety of wipes products specifically designed to be used on or for babies and small children which are often referred to as "baby-wipes."

While many of the wipes products referenced above are similar in their general design (for example a disposable substrate impregnated with an aqueous lotion) important differences exist. For example, it will be readily appreciated that a wipe designed for household cleaning and containing harsh ingredients is not suitable for use on skin such as for cleansing of one's face, hands, or buttocks. Even within a single sub-group of wipes ― such as baby-wipes ― products from even a single manufacturer may vary considerably to meet differing needs and desires of consumers. For example, baby wipes are currently offered with several variations of scent or perfumes or several lotion variations (such as versions for sensitive skin and non-sensitive versions). Other variations seen in baby wipes include different embossing patterns which may be included on the wipe substrate itself.

This proliferation of wipes offerings and of baby wipes offerings in particular does have its drawbacks, however. The sheer number of wipes offerings generally, and baby wipes offerings in particular, make it difficult for consumers to locate and purchase a desired product readily. This may be especially true in the case of baby wipes products where consumers often shop with babies or small children, resulting in less ability to intently focus upon various product offerings. Additionally, as wipes products and baby wipes products in particular are adapted to a wider variety of users, it may become increasingly difficult for consumers to self-select the product or products which most appropriately meet their needs. These needs could vary on the basis of such factors as desired benefit, use situation, and the stage of development of the baby who will be using the product.

In addition to proliferation of wipes products and baby wipes products generally, the market has also seen increased offerings surrounding the scenting of such wipes. Wipes are now offered in several scents including lavender, fruity scents, baby powder, and others. Some consumers either know of, or are instructed to use, choice of scent as a way of achieving a particular desired benefit (for example, some consumers choose lavender scented wipes because of the known correlations between lavender and calming responses). As scents and other elements of wipes are varied ― the number of offerings grows ever larger presenting an increasingly complex set of choices to the consumer. Many consumers are concerned about both purchasing the actual product desired as well as choosing the best-suited product for a particular intended use or desired benefit.

What is needed is an array or line up of products which clearly indicates to consumers which offering or offerings within the array are best adapted to correspond to a given desired benefit, use situation, or stage of development and which uses multiple sensory elements to achieve such correspondence. The correspondence of multiple sensory elements to one or more of a desired benefit, use situation, or stage of development can achieve both an enhanced use experience and an effective communication of the appropriateness of the tailored product to the particular consumer. An array or line up of baby wet wipes products specially tailored to a given consumer of group of consumers is needed. The present invention provides baby wet wipe products having multiple sensory elements specifically targeted to correspond to such desired benefit, use situation, and/or baby stage of development in response to these needs, although other advantages of may be present and other needs may also be addressed by the present invention described in detail below.

### SUMMARY OF THE INVENTION

The present invention is directed to a line up of wipes products. The line up includes at least a first wipe product and a second wipe product. Each of the first and second wipe products comprises a substrate and an aqueous composition impregnated into the substrate. Each of the first and second wipes products also comprises packaging containing the respective wipe product. The first wipe product comprises a first tailored scent element and a first tailored visual element. Likewise, the second wipe product comprises a second tailored scent element and a second tailored visual element. The first and scent elements differ from one another and the first and second visual elements differ from one another. The first wipe product is adapted to correspond on the basis of at least the first tailored scent element and the first tailored visual element to a first stage of baby development. The second wipe product is adapted to correspond on the basis of at least the second tailored scent element and the second tailored visual element to a second stage of baby development. Each of the first and second stages of baby development are different from one another. Each of the first and second wipe products is provided with indicia which communicates the stage of development to which the product is tailored.

The first stage of development may be selected from the group consisting of a bonding stage, an explorer stage, and a learner stage.

The first tailored scent element may comprise a scent selected from the group consisting of lavender, chamomile, rose, orange, tuberwose, sandalwood, cedarwood, bergamot, benzoin resin, and vanilla.

The second tailored scent element may comprise a scent selected from the group consisting of green tea, apple, ginseng, and menthol.

The first wipe product may include a first tailored tactile element and the second wipe product may include a second tailored tactile element. The first tailored tactile element and the second tailored tactile element may each be selected from the group consisting of molded substrates, rough surface packaging, smooth surface packaging, soft feeling packaging, and silky feeling substrates.

The first wipe product may include a first tailored auditory element and the second wipe product may include a second tailored auditory element. The first tailored auditory element and the second tailored auditory element may each comprise sounds selected from the group consisting of birds chirping, running water, leaves rustling, a frog sound, and music.

The first and second tailored visual elements may each comprise an image printed or embossed on the substrate.

The first and second tailored visual image may each comprise packaging images. These packaging images may be selected from the group consisting of a sleeping baby, a baby being held in the arms of an adult, and a natural scene involving water. The packaging image may involve a playing child.

The present invention may include a line up of wipes products including a first wipe product and a second wipe product each having a substrate and an aqueous composition impregnated into the substrate. Each product may be contained in packaging. The first wipe product may include a first, second, and a third targeted sensory element, each selected from the group consisting of a targeted scent element, a targeted visual element, a targeted tactile element, and a targeted auditory element. Similarly, the second wipe product may have a fourth, fifth, and sixth targeted sensory element each selected from the group consisting of a targeted scent element, a targeted visual element, a targeted tactile element, and a targeted auditory element. The first, second, and third sensory elements are each directed to different senses. The fourth, fifth, and sixth sensory elements are each directed to different senses. The first and fourth sensory elements are different from each other, but are directed to the same sense. Similarly, the fifth and second, targeted sensory elements are different from each other but are directed to the same sense. The sixth and third targeted sensory elements are different from each other but are directed to the same sense.

The first wipe product may be adapted to provide a soothing benefit, such adaptation including at least adaptation on the basis of said first targeted sensory element, said second targeted sensory element, and said third targeted sensory element.

The second wipe product may be adapted to provide a rejuvenating benefit, such adaptation including at least adaptation on the basis of said fourth targeted sensory element, said fifth targeted sensory element, and said sixth targeted sensory element.

### BRIEF DESCRIPTION OF THE DRAWINGS

It is believed that the present invention will be better understood in light of the detailed description below taken in conjunction with the accompanying drawings in which like numerals depict like elements and in which:
FIG. 1 is an example of a wipes package having artwork including targeted visual element.
FIG. 2 is an example of a wipes packaging having artwork including a different targeted visual element.
FIG. 3 is an example of a series of icons which may be used to represent various stages of development to which product offerings in a line up of the present invention may be tailored to correspond.

### DETAILED DESCRIPTION OF THE INVENTION

All express definitions of terms in this "Detailed Description of the Invention" are intended to be used to construe those terms for all purposes including for construction of the claims. Terms defined as being as used "herein" or for purposes of the present "invention", "application" or "specification" are intended to apply to the entire disclosure including the claims, and terms expressly defined are intended to have a consistent definition whenever used.

The present invention is directed generally to an array or a line up of wipes products. As used herein, the terms "line up" and "array" are intended to be interchangeable. An "array" or "line up" refers to two or more products which are each sold individually, but which are each part of a broader collective offering. The individual offerings in the line up or array are associated with each other by some commonality or connection. Examples of such a commonality or connection may include, but are not limited to: a common brand name, a common logo, a common color scheme, a common graphic, or any other indicia which signal that the products are related and are offerings to sub-groups within the overall group to which the line up or array as a whole is offered.

Users of baby wet wipes may comprise sub-groups each of which may have somewhat differing needs, desired benefits, or use situations. The present invention uses tailored product design features (specifically tailored sensory elements) to best meet (or correspond to) the particular needs, desired benefits or use situations of the various sub-groups. These tailored sensory elements communicate the reality of this tailoring (and therefore the appropriateness) of the product. In addition, this tailoring of sensory elements, by involving multiple senses in coordination, may constitute a powerful product feature in itself which delivers product benefits and use experiences superior to that of products which are not tailored in this manner.

Examples of the differing needs, desired benefits, or use situations for which individual products within the line up may be designed may include differing baby stages of development (for example, a baby in a bonding stage or a baby in a learning stage ― described in greater detail below). Other examples might include use in the nursery near nap-times, and use in active situations such as with meals or at playtime. Each of the examples given above may involve different needs from, or expectations of, baby wet wipes which differ from those in other examples.

As previously noted, the present invention is directed to line ups and arrays of wipes products. A wipes product may comprise at least a substrate. No particular design for such a wipe substrate is required and those of skill in the art will readily appreciate that all manner and variety of wipe substrates are suitable. Nonwoven materials are typical, though not required, for substrates. They may be formed into sheet-like material by any of a variety of well known processes including: airlaying, carding, spunbonding, spunlacing, hydroforming, and the like. The properties of a wipe substrate according to the present invention may be homogeneous or non-homogeneous. For example, while a single uniform web is suitable, it may be desired to have a layered substrate where fiber composition is different among some or all of the various layers. Additionally, zones of varying properties are also possible. Wipe substrate may be made from a variety of conventional natural and/or synthetic fibers including cellulosic based fibers (such as rayon, cotton, pulp, lyocell, and variations of these), or polyethylene, polypropylene, bicomponent or other synthetic fibers.

Wipes suitables for use in line ups of the presented invention may be provided in a wide variety of convenient shapes and sizes although there is no particular shape or size which is necessary. For ease of manufacture and dispensing, rectangular sheets of wipe material may be used, although wipes can also be rounded, circular, or other shapes. Individual sheets can be separately packaged (such as in an interleaved "pop up" configuration) or the wipes can be packaged in a continuous stack or roll to be separated later by the user (e.g., by perforations or the like).

For ease of illustration and explanation the present invention will be described with reference to wet wipes and baby wet wipes in particular. While these examples are used for purposes of demonstration in this application, line ups and arrays of the invention may be practiced in other wipes contexts outside of wet wipes and baby wet wipes. As used in this application the term "wet wipes" means a wipe product which has a moisture content of greater than about 10% by weight of the substrate. Some wipes products are delivered in an apparent "dry" state although they have measurable moisture content. For example, some cleansing wipes (such as for bathing, face washing, or makeup removal) have a low moisture content (typically less than about 10%) cleansing solution which is impregnated or otherwise integrated with the wipe substrate. This solution may be released from the wipe upon immersing the wipe in water. Wet wipes often have higher moisture content as packaged, such as moisture content in the range from about 50% to about 350% or more by weight. For purposes of this application, the term "wet wipe" will refer to a wipe product having a moisture content greater than about 10% moisture by weight of the substrate. For purposes of this application the moisture content imparted into the substrate will be referred to generically as "lotion." Such lotions may be solutions, emulsions, or any other type of composition, and are typically some sort of aqueous cleansing formulation. A wide variety of such lotions is known in the art and is suitable.

Baby wet wipes are typically used by consumers for cleaning of the skin of babies and children. As used herein, the term "babies" is intended to describe persons ranging in age from newborn to about 2-7 years old. Young children often referred to variously as toddlers, pre-school aged children, or even school aged children all come within this definition. It is specially contemplated that the definition of babies as used herein will encompass children of an age who can talk and have greater dexterity, motor skills and the like than younger babies (for example, as compared to newborn babies). Such older babies (as the term is used herein) may be able to use baby wet wipes products themselves while younger babies may need a caregiver to use the product on them. There is no single upper age limit for babies (as that term is used herein) and it will be recognized that babies progress through stages of development at differing rates. The characteristics of the stages and situations (described in greater detail below) may be more significant than the typical age ranges associated with such stages and situations. Age ranges are by necessity averages and generalities. The ability of the line ups of the present invention to target on the basis of situation and stage (as determined without strict reference to age) is one of its benefits. While no particular upper age limit exists for babies, it is typical that those making the ultimate purchase decision with respect to particular products in the line up will not be a baby within the meaning defined above. In other words, even though older babies may be involved in the use and purchase of baby wipes for their stage or use situation, they often will not make the entire purchase decision on their own (as an example, a 5 year old might tell his or her parent what product he or she desires, but ultimately the parent makes the decision to purchase or not). The significance of this will become more apparent in the context of the discussion below about targeting sensory elements in the line up and the communication of such targeting to both the baby and the caregiver.

Typical uses of baby wet wipes products include cleaning of the buttocks and perineal area during a diaper change, cleaning of the hands, face, and other body parts. The terms "baby wet wipes" and "wet baby wipes" are interchangeable and are meant to encompass wet wipe products which are specifically designed and targeted to be used by children ranging in age from newborn to toddlers and children in the 2-7 year old range. Such wipes for babies in the upper end of these age ranges are sometimes referred to as "toddler wipes" or "toddler toilet wipes" or by a similar designation. An example of such a product is the PAMPERS KANDOO wipe manufactured and sold by the Procter and Gamble Company of Cincinnati, Ohio. Even if sometimes commercially or informally referred to by a designation of other than "baby wipes" such products are expressly intended to be included within the definition of baby wet wipes as that term is used herein.

A line up of baby wet wipes according to the present invention may comprise two or more targeted or adapted individual baby wet wipe offerings. As used herein, the terms "targeted," "tailored," and "adapted to correspond" are meant to be interchangeable and are taken to mean that aspects of the products are specifically chosen to correspond to one or more aspects of a sub-segment of the baby wet wipes market as a whole. In particular, targeted or tailored elements are different among product offerings in the line up on the basis of some aspect or attribute of the sub-population of baby wet wipes users to which the element is being targeted or adapted. For examples, individual baby wet wipes products in a line up of such products according to the present invention may be targeted to two or more baby stages of development, two or more use situations, two or more desired benefits, or any combination of these. For ease of understanding of these concepts and the concepts of targeted or tailored offerings generally, a more complete description of baby stages of development will be provided for illustrative purposes.

The term "baby stages of development" refers to the main characteristics that are typical for babies as they progress in their development from newborns to babies in the older age ranges (such as 2 to 7 years old). In particular, in the context used herein, each stage of development (described in greater detail below) is characterized by representative cognitive, motor, emotional, and learning developmental attributes. Individual wet baby wipes in line ups of the present invention may be tailored to be particularly well suited (compared to baby wipes products not so tailored) to meet the needs of, or enhance the sensory experience of babies in that stage as compared to baby wet wipes generally or baby wet wipes tailored for another stage.

The baby stages of development may range from newborns to active toddlers seeking independence. For instance, a first stage of development might cover a prelocomotive phase and include newborns and other immobile infants in a bonding stage with a parent or caregiver, such babies may have a level of activity which might include little more than head raising or rolling over. A second stage of development might cover a discovering stage comprising a crawling phase and include curious toddlers developing activity in the form of sitting and mobility in the form of scooting, rolling and crawling. A third stage of development might cover an exploring stage comprising a walking phase and include toddlers whose level of activity includes standing, walking and beginning to run. A fourth stage of development might cover a learning phase and include toddlers capable of doing things by themselves such as dressing and developing coordination which enables them to walk and run without losing balance. A fifth stage of development might cover a training stage and include toddlers undergoing toilet training, attempting to achieve independence. Other stages are contemplated. A more detailed description of stages of development of babies may be found in U.S. Patent 6,763,944. It is also possible, if desired, to combine some of these stages in given executions or to sub-divide them further. For example, a wipes line up tailored to babies in different stages might include an offering for babies in a first bonding stage, an offering for babies in a second exploring stage, and an offering for babies in a third learning stage. In this particular example, the exploring stage could be a combination of the second discovering and third exploring stage described above and in U.S. Patent 6,763,944. Similarly, the learning stage may represent a combination of the fourth learning stage and fifth training stage described above and in U.S. Patent 6,763,944.

The attributes important to tailoring baby wipes offerings to the various stages of development may differ from those important to tailoring of diapers or other baby products to corresponding stages. In other words, the relevant characteristics of a given stage (such as the bonding stage) of development to which a baby wet wipe can be best designed will differ from those relevant characteristics of the bonding stage to which a diaper product (or other baby product) can be best designed. Additionally, individual baby wipes products in a line up of the present invention may be tailored on the basis of subsegments of babies which are not stages per se. Examples of such can include use situations and/or benefits. Either of both of these examples can be delivered in a tailored line up of wipe products on their own or in combination with stage of development tailored products.

An example of a line up of wipes products which is tailored on the basis of desired benefit might include a line up which includes a wipe product tailored to provide a soothing benefit to the baby and a second wipe product tailored to produce a stimulating benefit to the baby. The choice of product tailored for the soothing benefit could be made on the basis of situation (for example the stimulating product may be desired during active periods such as playtime, in the playground, at meals, etc.).

It is also possible to tailor wipes products in the line up to both stages and desired benefit at the same time. In other words, the sub-segment of users to which a given offering in the line up is tailored could include more than one variable.

One example of a line up of the present invention with a tailored offering for each stage of development might include an offering tailored for a bonding stage. Such a wipe product could be specially designed to incorporate sensory elements which convey softness and warmth, advanced skin care, and gentle ingredients. Optionally, sensory elements designed to produce a soothing benefit may be desired either as part of tailoring to this stage or independently of it. An offering tailored to the explorer stage might incorporate sensory elements designed to be appropriate to the developing senses of babies in this stage. This could include sensory elements which are vibrant and fresh, and stimulating. Optionally, sensory elements designed to produce a stimulating benefit may be desired either as part of tailoring to this stage or independently of it. An offering tailored to the learning stage might incorporate sensory elements designed to be appropriate to promoting the empowerment and independence of babies in this stage. This could include sensory elements which are easy, fun and intuitive for babies in this stage to use. The sensory elements could be chosen to promote teaching, to be fun, or be inventive or different for the babies in this stage.

Other examples of sub-groups to which wipes products in lines ups of the invention could be tailored include those desiring a deep cleaning experience and those desiring an experience tailored to sensitive skin. Sensory elements of such offerings can be designed to reinforce these experiences.

One manner in which the various wipes offering in an array or line up of the present invention can be tailored is on the basis of sensory elements. As described above, the various product offers can be tailored to correspond to babies at different stages of development. The stages of development in this example represent the sub-groups for whose needs or characteristics the sensory elements are tailored to correspond to while the sensory elements represent what is tailored to better meet the referenced needs or characteristics.

Sensory elements of wet wipes products which may be tailored in line ups of the present invention may include any or all of scent elements, visual elements, tactile elements, and auditory elements. Each of these elements is designed to be perceived by a given sense such as smell, sight, touch, and hearing, respectively. In well executed line ups of the present invention multiple sensory elements are both provided and tailored in accordance with the present invention.

As noted previously, wet wipes, and baby wet wipes in particular are often provided with a scent element. Scents for wet baby wipes may be incorporated into the lotion. A variety of scents are already provided to a currently available wet baby wipes. Currently available scents include lavender scents, chamomile scents, fruity scents, and baby powder. Other scents beyond those currently available may also be provided as part of the present invention. The lotion of the wet baby wipe may comprise an effective amount of a perfume or other scent delivery ingredient in order to deliver a desired amount of a scent to the wipe. Scented wipes may be desired to mask odors which may otherwise be associated with use of the wet baby wipe. These odors could include the odor of the lotion itself or the odor of bodily waste being wiped from the baby or child. Beyond masking odor, another purpose of providing scent to a wet baby wipe could include providing a pleasant experience in using the wipe through scent. Such a scent experience could be provided for its own sake, or could be provided in order to deliver some benefit.

The use of scents to deliver some benefit has gained popularity in recent times. It has been known that various scents are correlated with various physiological responses and other potentially desired reactions or benefits. The field of aromatherapy is the application of these correlations between scents and particular responses. Examples of some scents which are correlated to a particular physiological response are the use of scents to produce a soothing response. Scents which may be used to elicit a soothing response may include lavender, chamomile, tuberose, sandalwood, cedarwood, bergamot, cinnamon, vanilla, benzoin resin, valerian, hops, juniper, ylang ylang, anethole, 2-ethyl-4(2',2',3'-trimethylcyclopent-3'-enyl)but-2-enol (eg Bangalol®), basil oil, cis-hex-3-enol, coumarin, ethylene brassylate, ethyl linalol, 2-(2'-methylpropyl)-4-hydroxy4-methyltetrahydropyran (eg Florosa®), hexahydro-4, 6,6,7,8,8-hexamethylcyclopenta[g]-2-benzopyran (eg Galaxolide®), geraniol, cyclohexadecanolide, cyclopentadecanone, methyl anthranilate, alpha-iso- methyl ionone, mixtures of dimethyl benzyl carbinyl butyrate and phenoxyethyl isobutyrate (e.g. Prunella®), mixtures of cyclohexadecanolide and cyclopentadecanone (e.g. Silvanone®), alpha- terpineol, 6-acetyl-1-isopropyl-2,3,3,5-tetrahydrotetralin (eg Traseolide®), 2-ethoxy-4-methlyphenol (eg Ultravanil®), gamma-undecalactone, vetiver oil and vetiver acetate. Another example of a physiological response which may be elicited from the use of scents is a stimulating response. Scents which may be used to elicit a stimulating response include green tea, citrus (such as orange), apple, ginseng, menthol, mints (such as peppermint), caraway, clary sage, coriander, eucalyptus, grapefruit, lemon, lemongrass, lime, petit grain, sage, tea tree, cardamom, cedarwood, dill, frankincense, geranium, jasmine, neroli and rosemary. Other scents which may be provided in a baby wet wipe may include honey, rosewater, witch hazel, white tea, licorice, anise, apricot kernel, avocado, balm mint, bee balm, birch, bitter almond, bitter orange, clay, cloveleaf, clove, coconut, cypress, evening primrose, fennel, gardenia, ginger, grape seed, hazelnut, hyptis, indigo bush, jojoba, kiwi, laurel, linden, lovage, matricaria, musk rose, nutmeg, olibanum, orange flower, orange, peach kernel, patchouli, pennyroyal, pine, pine tar, rose hips, rose, rue, sambucus, sassafras, sesame, silver fir, sweet almond, sweet marjoram, sweet violet, tar, thyme, wheat germ, wild mint, yarrow and the like, as well as mixtures thereof. It is not necessary to provide a scent element to a wipe in order to elicit a particular physiological response or other benefit. Scent elements may be provided to a wipe for the appeal of the scent itself. For purposes of this application, the absence of scent or a neutral scent is considered within the definition of a scent element. This is especially the case where a neutral scent or the absence of a scent is specifically chosen to correspond to one or more of a targeted desired benefit, use situation, or stage of development.

It has been found during development of the present invention, that targeting of a particular scent or scent types to a particular sub-set of consumers may increase the benefit or acceptance of the wipe product itself. For example, the examples given above where scent elements are tailored to elicit either a soothing response or a stimulating response may be provided independently or in combination with tailoring for stages of development. For example, a baby wet wipe having a lavender scent may be particularly well suited for use either by bonding stage babies, by more fussy babies or children, for use prior to nap or bedtimes, or any other time a calming experience is desired. This example illustrates that the targeting of a particular scent (e.g. lavender) to a particular sub-group of consumers (e.g., those desiring a soothing experience) may be done on the basis of situation (e.g., a child getting ready for bed) or stage of development (e.g. young babies who sleep more and may desire a soothing nurturing environment). As another example, a stimulating scent (such as green tea or citrus) may be targeted to more active children (such as babies in an explorer stage of development) in an exploring stage of development. Similarly, such targeting may be on the basis of situation, such as children who are playing ― a caregiver may want to use the wet baby wipe to wipe such a child's hands and face, but not elicit the soothing response a lavender scent might. Indeed in such an example, a rejuvenating response may be desired.

In addition to situational targeting, scent elements in wet baby wipes may be targeted on the basis of scent preference. It has been found during development of the present invention that scent preference may be correlated to age or stage of development of babies and children. For example, a product targeted for a bonding stage might include a scent element which conveys the perception of gentleness, calming, or caring for the skin. Such scent elements may include floral scents, other botanically based scents, mild or other gentle scents, or any of the scents identified above in connection with a soothing response.

Learning stage babies may be provided with a product including a fruit based scent. Learning stage babies may even tend to refer to such scents using food names such as "grape" or "strawberry." Other scents which may be suitable for learning stage babies are citrus based scents, scents which are vibrant and stimulating, including any of those identified above as providing a rejuvenating benefit.

It has been found during development of the present invention that not only may it be desirable to target scent elements to specific groups of consumers as described by examples above, but it may also be desirable to combine such scent element target with other sensory element targeting. For example, it has been found during development of the present invention that it may be desirable to combine targeting of scent elements in wet baby wipes with visual elements, tactile elements, and/or auditory elements in wet baby wipes. Without wishing to be bound by theory, such an approach is believed to offer various possible benefits. Firstly, as scent (and other product variations) offers become more numerous and more specific, the ability of a given consumer or group of consumers to locate readily the product desired becomes complex. While a written description of the scent on the packaging of the product (such as an indication that the wipe is a lavender scented wipe) may be useful, such labeling is not always readily discerned when viewed quickly among a multitude of product offerings. A targeted visual element (or other sensory element) chosen to compliment the targeted scent element may enhance the ability of the relevant sub-group of consumers readily to locate the product on the shelf and even at home. Additionally, it has been found during development of the present invention that the combination of multiple senses (such as smell and sight) may provide an overall more positive use experience of a wet baby wipe and the various sensory elements can reinforce and even provide a multiplied benefit. For example, if a given baby wet wipe product is tailored to provide a soothing benefit, the combination of a soothing sensory element (such as a lavender scent) with a soothing visual element and soothing tactile element may all make finding the desired product (i.e. a soothing benefit baby wipe) easier and may enhance the soothing over just a soothing scent element or a soothing visual or tactile element alone and may provide a synergistic effect in combination. In this sense the multiple senses with which the sensory elements interact reinforce and compliment each other. Such reinforcement may even lead to stimulations of the baby's sensory development appropriate to the targeted stage.

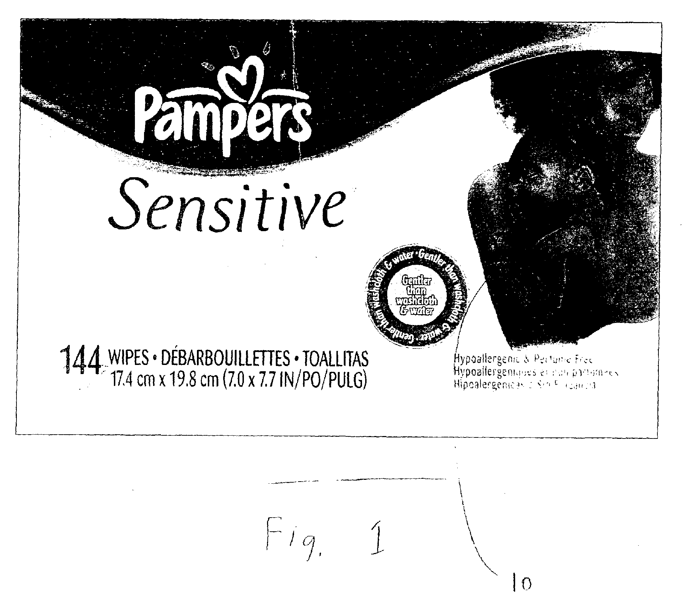

A visual element targeted to a particular benefit, use situation or stage of development can take many forms. For example, a visual element tailored for soothing could comprise a scene (such as a drawing, photograph, or icon) tailored to produce a soothing response or elicit an identification with a calm scenario in the viewer. Examples of such calm imagery could include an image of sleeping baby, or of a calm or sleeping baby being held in a mother's arms. An example of such an image which could serve as this type of targeted visual element is shown as image 10 in FIG. 1. A soothing natural scene such as a peaceful-looking field or water-including image could also be used as a visual element tailored for soothing. In addition to scenery (such as packaging scenery), a targeted visual element could be provided in the form of color. Soothing or otherwise calm suggesting colors (such as muted tones, lack of sharp contrast, pastels, etc.) may produce a visual element tailored to soothing. Out of focus or muted or rounded graphics or packaging edges could also be used. Icons of a sleeping baby might be provided on the packing or on the wipe itself to communicate visually a soothing benefit in this example. Such soothing visual elements may be provided for the sake of tailoring to a soothing response or may be appropriate as part of a wipe product tailored for babies in the bonding stage. Visual elements tailored to the bonding stage are characterized by soft and warm colors, tones, and imagery. A tailored visual element might include a picture, drawing, or photograph representing one or more ingredients in the wipe ― for example, a picture of a lavender flower if a lavender perfume is an ingredient in the wipe product.

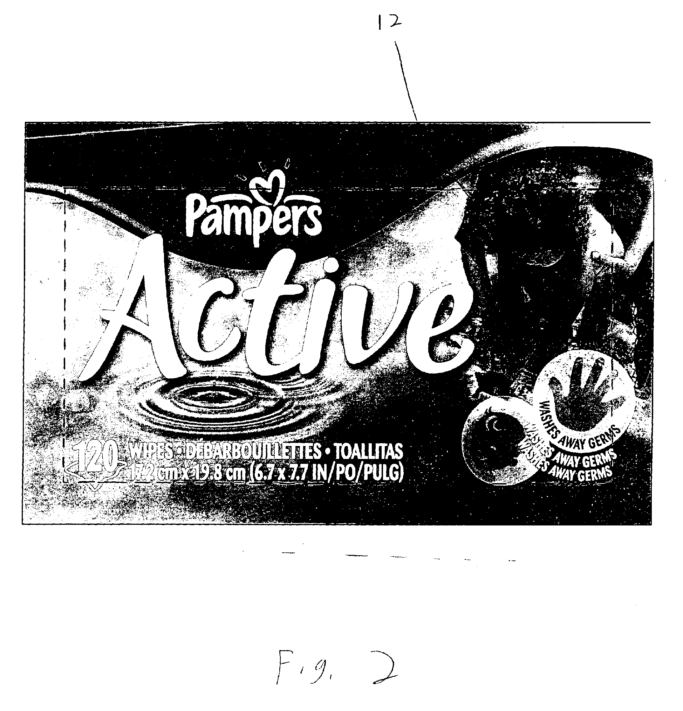

By way of another example, if the visual element were to be tailored to a rejuvenating benefit, different visual elements would be appropriate. Examples of such could include a photograph, icon, or drawing depicting more active or stimulating scenery. This may include an image of a child playing, exploring, or otherwise interacting with others or with the environment in an active manner. An example of such an image which could serve as this type of targeted visual element is shown as image 12 in FIG. 2. Colors in such a visual element may be vibrant, bright, or have higher contrast than those tailored for soothing. Primary colors, shaper graphics, complex three dimensional shapes and patterns, and crisper packaging edges might all be examples of stimulating visual elements. The packaging or wipes themselves might be provided with icons of a walking or playing baby or of a hand or baby face. Such icons would convey visually a rejuvenating benefit or active situation (such as the wipe is tailored to wash hands and face during playtime). These rejuvenating features may be provided for the sake of tailoring to such a response, or may be part of a wipe product tailored for babies in the exploring stage.

It should be noted that not all babies in the bonding stage will desire a soothing experience for all situations in baby wipes will be used. However, soothing is a characteristic want of the stage. It may be recognized in well designed line ups of the present invention that while soothing is a typically desired feature of products tailored to the stage, there may be use-specific instances where even babies in the bonding stage may desire a seemingly diametrically opposed benefit (such as rejuvenating). In such a case, a well designed line up could communicate the suitability of such products for the typical use associated with the stage (e.g., a bonding stage designed wipe) while also communicating use specific situations which might call for the more appropriate selection and use of a different offering in the line up.

Tailored visual elements may be provided on the packaging of the wipes or on the wipes themselves or both. Tailored visual elements may be provided in combination with other tailored sensory elements such as the scent elements described above and the auditory and tactile elements described in more detail below. For examples, a baby wet wipe tailored for use with bonding stage babies may have a scent element tailored for such babies as noted above (such as a botanical-based scent) and a visual element tailored for such babies (such as high contrast colors like red and black). A baby wet wipe tailored for use with learning stage babies might have a tailored scent element such as a fruity scent. A corresponding tailored visual element might include character graphics of popular children's characters, puzzles, or other elements more suitable for the learner stage ― for example, by being interesting and engaging to the child itself.

Other targeted sensory elements may also be provided as part of the present invention. For example, targeted auditory elements, targeted tactile elements, or both could also be provided. Examples of tactile elements include texturing of the wipe substrate itself. These can be varied to be highly textured or smooth or any other tactile feel desired. Other tailored tactile elements could be provided in association with the packaging. For example, the wipes packing could be made from a soft-feeling and/or flexible material or could be made from a more textured surface. Such surfaces could be chosen to reinforce other sensory elements (such as visual elements or scent elements). Tactile elements on the packaging or wipe substrate itself could be tailored to stages of baby development. For example, a highly molded or textured substrate (such as described in U.S. Patent Application 2004/0242097) may be particularly suited for development of tactile sensation associated with the exploring stage. A soft tactile feel to the packaging and the substrate itself may be desirable in the bonding stage. Tactile elements of both the substrate and packaging could be used to both deliver and reinforce a deep cleaning benefit which may be a desired situational basis for targeting. Tactile elements can also be targeted by varying the thickness of the substrate. Such variations can convey a feel of softness, deep cleaning, stimulate the sense of touch, or achieve any other tactile response appropriate to the targeted stage of development or situation. The size of the wipe itself can also be part of the basis of a tactile element particularly in the learner stage where children are more likely to use the wipe themselves. The proper selection of wipe size for the size of a learner stage baby's hand can be part of the tactile experience associated with such a wipe.

Auditory elements can also be included as part of the present invention. The packaging of a wet baby wipes product can be provided with a sound generation mechanism. An example would be a chip which produces a sound when a wipe tub is opened for dispensing, when a wipe is pulled through the orifice, when the tub is reloaded, or other suitable event. Such sounds could be tailored to compliment the scent, visual, or tactile elements which may be provided in a given offering. For example, a baby wet wipe with a targeted scent element (such as botanical scent for the bonding stage) can be packaged in a tub with a photograph of a sleeping baby in its mother's arms (a targeted visual element). These elements could be combined with a device which emits the sound of gently running water when the package is opened. Of course in this particular example, any other calming or soothing sound is an appropriate targeted auditory element. Other sounds could be targeted to other situations or stages of development. Examples include a "fanfare" type sound for a rejuvenating targeted wipe product, a "croaking" or "rabbit" sound for packaging having tailored characters (such as a frog character) for toddlers and pre-school aged children, or any other suitable targeted auditory element.

The various sensory elements can be included into the wipes products in any suitable manner. For example, scent elements of the present invention may be provided to the wipe product in any of a variety of ways. For example, the lotion of the wipe may incorporate a scent (such as by way of perfume) such as is conventional today. Additionally, the scent element of a wipe product may be provided other than by way of the lotion. For example the fibers of the wipe substrate itself could be scented (such as being formed out of scented resin in the case of a synthetic fiber) or by being scented upon formation of a web in the case of natural fibers. Delivery of scents though package inserts (such as a slow-release scent element) packaged in the interior of a wipes container or wrapper, encapsulated scent features, or the like are also possible. For example, a wipe tub could be fitted with a ring or other structure near a dispensing orifice which either releases a scent or scents the wipe as each wipe is pulled through the orifice for dispensing.

Visual elements of the present invention may also be provided in any suitable manner. The visual element may be provide on the packaging (or as an aspect of the packaging), on the wipe itself, or both, or otherwise associated with the wipe. Visual elements could include printing, colors, embossing or the like. The finish of the wipe or packaging (such a highly reflective or matte finish or a bright white color versus a beige or less reflective color) could be aspects of visual elements.

As will be appreciated by those of skill in the art in light of the above disclosure, the various sensory elements described above can be combined and utilized in a variety of permutations. Ideally, such combinations are chosen so that a particular product combines multiple sensory elements and so that the sensory elements compliment each other. The products may be offered as an array with common line up elements (such as common advertising, common packaging elements, common shelving elements).

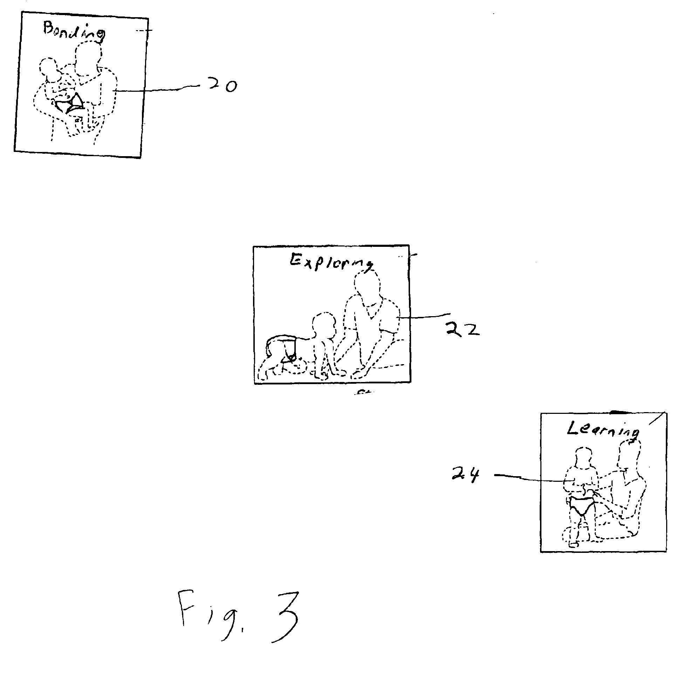

In well executed line ups of the present invention the various offerings in the line up may be expressly coordinated with each other though some sort of common indicia or indication. For example, a common set of icons representing the entire line up and the position of a particular offering within the line up could be employed. An example of such a set of icons is shown in FIG. 3. Icon 20 may represent the bonding stage of development, a second icon 22 may represent the exploring stage, while a third icon 24 may represent the learning stage. Each product in the line up may be provided with packing which depends each of the icons in FIG. 3 with the relevant icon (i.e., the one corresponding to the stage to which the product is tailored) being larger, highlighted, or being identified as the relevant icon among the set. Such an icon system both communicates what the particular tailored product is adapted to correspond to (e.g., a given stage of development) as well as what it is not corresponding to (e.g., the other stages available). Both the positive and negative aspects of such a communication may assist purchasers in making choices of product better adapted to their product needs.

A line up of the present invention could include common color schemes with variations representing the various targeted offerings. Such variations could be consistent with other baby products tailored to stages. For example, the wipes products of the present invention could be offered as an array consistently with any of the ways in which an array of baby stage tailored diaper products such as those described in U.S. Patent 6,763,944. Line ups of the present invention can also be provided with ancillary aides, tools, or selection mechanisms or devices to assist consumers in selected the appropriate product in the line up for their needs. For example, interactive displays including charts, wheels, kiosks, computer terminals, booklets, flyers or the like could describe attributes of stage of development and assist consumers in determining which stage their baby is in, and make a corresponding product recommendation within the lineup. Such guides could be offered on the package of a given product, otherwise on or near the shelf, or even removed from the point of purchase (such as on a web-site, telephone system, or through print media).

A line-up or array configuration of the present invention may be arranged so as to allow consumers to see the various offerings quickly and easily and readily understand the various tailored aspects of the product particularly with respect to tailored aspects of the other products in the line. In other words, the impact of a wipe with elements which may be most desired by babies in the bonding stage may be diluted if the consumer does not know that such a product is so tailored and can contrast such tailoring with a product tailored to the exploring stage having different tailored sensory elements. The ability to spot the tailoring in the context of the larger line up increases both the impact and efficacy of the tailoring of each individual product offering.

A potential advantage of tailoring across sensory elements is that some consumers and users will prefer certain sensory elements over others. Additionally, the combination of sensory elements may have synergistic effects which the provision of single sensory elements may not deliver.

While particular embodiments of the present invention have been illustrated and described, it would be obvious to those skilled in the art that various other changes and modifications can be made. It is therefore intended to cover in the appended claims all such changes and modifications.

## Claims

1. A line up of wipes products comprising:
a first wipe product, said first wipe product comprising a substrate, an aqueous composition impregnated into said substrate, and packaging which contains said first wipe product, said first wipe product further
comprising
a first tailored scent element, and
a first tailored visual element; and
a second wipe product, said second wipe product comprising a substrate, an aqueous composition impregnated into said substrate, and packaging which contains said second wipe product, said second wipe product
further comprising
a second tailored scent element, and
a second tailored visual element;
said first tailored scent element differs from said second tailored scent element, and said first tailored visual element differs from said second tailored visual element, and wherein said first wipe product is adapted to correspond on the basis of at least said first tailored scent element and said first tailored visual element to a first stage of development, and said second wipe product is adapted to correspond on the basis of at least said second tailored scent element and said second tailored visual element to a second stage of development, wherein each of said first stage of development and said second stage of development differ from one another, and wherein each of said first wipe product and said second wipe product is provided with indicia which communicates the stage of development to which said product is tailored.

2. The line up of claim 1 wherein said first stage of development is selected from the group consisting of a bonding stage, an explorer stage and a learner stage and wherein said second stage of development is selected from the group consisting of a bonding stage an explorer stage and a learner stage.

3. The line up of any of the preceding claims wherein first tailored scent element comprises a scent selected from the group consisting of lavender, chamomile, rose, orange, tuberose, sandalwood, cedarwood, bergamot, benzoin resin, and vanilla.

4. The line up of any of the preceding claims wherein said second tailored scent element comprises a scent selected from the group consisting of green tea, apple, ginseng, and menthol.

5. The line up of any of the preceding claims wherein said first wipe product further comprises a first tailored tactile element and said second wipe product further comprises a second tailored tactile element, and wherein said first tailored tactile element and said second tailored tactile element are each selected from the group consisting of molded substrates, rough surface packaging, smooth surface packaging, soft feeling packing, and silky feeling substrate.

6. The line up of any of the preceding claims wherein said first wipe product further comprises a first tailored auditory element and said second wipe product further comprises a second tailored auditory element, and wherein said first tailored auditory element and said second tailored auditory elements comprises sounds selected from the group consisting of birds chirping, running water, leaves rustling, a frog sound, and music.

7. The line up of any of the preceding claims wherein said first tailored visual element and said second tailored visual elements each comprises an image printed or embossed on said substrate.

8. The line up of any of the preceding claims wherein one or both of said first and said second tailored visual elements comprises a packaging image, preferably said first visual element being selected from the group consisting of a sleeping baby, a baby being held in the arms of an adult, and a natural scene involving water, and preferably said second tailored visual element comprises a packaging image involving a playing child.

9. A line up of wipes products comprising:
a first wipe product, said first wipe product comprising a substrate, an aqueous composition impregnated into said substrate, and packaging which contains said first wipe product, said first wipe product further comprising
a first targeted sensory element selected from the group consisting of
a targeted scent element, a targeted visual element, a
targeted tactile element, and a targeted auditory element; a second targeted sensory element selected from the group consisting of
a targeted scent element, a targeted visual element, a
targeted tactile element, and a targeted auditory element; a third targeted sensory element selected from the group consisting of
a targeted scent element, a targeted visual element, a
targeted tactile element, and a targeted auditory element;
wherein each of said first, said second, and said third targeted sensory elements is directed to a different sense than each of the others; and a second wipe product, said second wipe product comprising a substrate, an aqueous composition impregnated into said substrate, and packaging which contains said second wipe product, said second wipe product further comprising
a fourth targeted sensory element different from said first targeted sensory element, but directed to the same sense;
a fifth targeted sensory element different from said second targeted sensory element, but directed to the same sense;
a sixth targeted sensory element different from said third targeted sensory element, but directed to the same sense.

10. The line up of claim 9 wherein said first wipe product is adapted to provide a soothing benefit, such adaptation including at least adaptation on the basis of said first targeted sensory element, said second targeted sensory element, and said third targeted sensory element, and wherein said second wipe product is adapted to provide a rejuvenating benefit, such adaptation including at least adaptation on the basis of said fourth targeted sensory element, said fifth targeted sensory element, and said sixth targeted sensory element.
